# FASCICULE DE BREVET EUROPEEN

(11) **EP 1 072 586 B1**
(45) Date de publication et mention de la délivrance du brevet: **13.11.2002**
(21) Numéro de dépôt: 00401653.1
(22) Date de dépôt: 13.06.2000
(51) Int. Cl.: C07C 303/44, C07C 309/04

(54) **Purification d'acides alcanesulfoniques**
Reinigung von Alkansulfonsäuren
Purification of alkanesulfonic acids

(30) Priorité: 27.07.1999 FR 9909712
(43) Date de publication de la demande: 31.01.2001
(73) Titulaire: Atofina, 92091 Paris La Défense Cedex (FR)
(72) Inventeur: Gancet, Christian, 64140 Lons (FR)
(74) Mandataire: Leboulenger, Jean

(56) Documents cités:
- FR-A- 2 305 429
- GB-A- 1 264 293
- US-A- 3 496 224
- US-A- 5 028 736
- US-A- 5 434 301
- PATENT ABSTRACTS OF JAPAN vol. 010, no. 208 (C-361), 22 juillet 1986 (1986-07-22) & JP 61 050957 A (DAIICHI SEIMO KK), 13 mars 1986 (1986-03-13)

## Description

La présente invention concerne le domaine des acides sulfoniques et a plus particulièrement pour objet la purification des acides alcanesulfoniques tels que l'acide méthanesulfonique (AMS) en vue d'en réduire la teneur en acide sulfurique.

Les acides alcanesulfoniques et, plus particulièrement, l'AMS sont utiles comme catalyseurs d'estérification et pour le placage de métaux conducteurs.

La synthèse de ces acides par oxydation du thiol correspondant, par hydrolyse d'un halogénure d'alcanesulfonyle ou encore par oxydation du diméthyldisulfure génère diverses impuretés dont la présence peut s'avérer gênante lors de l'utilisation. C'est en particulier le cas pour l'acide sulfurique qui peut se trouver dans l'AMS à des concentrations allant de quelques centaines à quelques milliers de ppm et dont la présence est néfaste dans l'utilisation de l'AMS pour le placage des métaux conducteurs; pour cette application, la spécification commerciale demande une teneur en acide sulfurique inférieure à 150 ppm.

Contrairement à d'autres impuretés comme l'acide chlorhydrique, l'acide sulfurique ne peut pas être éliminé par strippage. Différentes méthodes d'élimination de l'acide sulfurique sont connues, mais qu'elles soient physiques (cristallisation fractionnée ou séparation sur membrane de nanofiltration) ou chimiques (précipitation de sulfates de métaux alcalino-terreux, réduction électrochimique sélective de H₂SO₄ ou réduction sélective de H₂SO₄ par l'hydrogène sulfuré), aucune de ces méthodes ne donne de résultat satisfaisant.

Il a maintenant été trouvé qu'on peut réduire considérablement la teneur en acide sulfurique d'un acide alcanesulfonique par mise en contact de ce dernier avec une résine basique échangeuse d'anions.

L'homme de l'art sait que les résines basiques, en particulier les résines basiques fortes, sont capables de fixer les différents anions avec une affinité variable qui dépend de la nature de l'anion considéré et du nombre potentiel de charges susceptibles d'être portées par cet anion.

Il est, par contre, surprenant qu'une résine basique échangeuse d'anions puisse fixer sélectivement l'acide sulfurique, présent en faible concentration, dans un acide alcanesulfonique (par exemple l'AMS) très concentré. En effet, on pouvait s'attendre à ce qu'une forte concentration en acide alcanesulfonique empêche toute sélectivité de s'exprimer et cela, d'autant plus, que dans ce milieu très acide, l'acide sulfurique n'est probablement pas ionisé et doit donc être échangé avec le contre-ion présent sur la résine, dans des conditions a priori peu favorables. US-A-3 496 224 concerne la purification des acides organiques sulfaniques avec une résine acide échangeuse de cation.

L'invention a donc pour objet un procédé de purification d'un acide alcanesulfonique pour en réduire la teneur en acide sulfurique et accessoirement celle d'anions autres que l'anion alcanesulfonate, caractérisé en ce qu'il comprend au moins une étape de mise en contact d'une solution aqueuse de l'acide alcanesulfonique à purifier avec une résine basique échangeuse d'anions.

Bien que le procédé selon l'invention vise en premier lieu la purification de l'AMS, il peut s'appliquer aussi à celle de tous les acides alcanesulfoniques miscibles à l'eau, notamment les acides contenant jusqu'à 12 atomes de carbone et, plus particulièrement, ceux contenant jusqu'à 4 atomes de carbone comme l'acide éthanesulfonique, l'acide n-propanesulfonique et l'acide n-butanesulfonique.

Dans la solution aqueuse d'acide alcanesulfonique à purifier, la teneur pondérale en acide alcanesulfonique peut varier dans de larges limites (10 à 90 % selon la nature de l'acide), mais elle est avantageusement comprise entre 60 et 80 % et le plus souvent voisine de 70 % (teneur habituelle des solutions commerciales d'AMS).

Les résines basiques échangeuses d'anions à utiliser pour la mise en oeuvre du procédé selon l'invention sont bien connues et disponibles dans le commerce. On peut utiliser des résines basiques faibles comme celles porteuses de groupements fonctionnels amine secondaire (par exemple dialkylamino), mais on préfère mettre en oeuvre des résines basiques fortes comme celles porteuses de groupements fonctionnels ammonium quaternaire, en particulier de groupements -N^{⊕}R₃ où R est un radical alkyle en C₁ à C₄, de préférence méthyle. Ces groupements fonctionnels sont généralement fixés sur un copolymère polystyrène-divinylbenzène à structure macroporeuse. Les résines échangeuses d'anions préférées selon l'invention sont celles commercialisées sous la dénomination DIAION® HPA25 par la Société Resindion et sous la dénomination AMBERLITE® IRA92 par la Société Rohm & Haas.

Ces résines ne sont généralement pas très stables thermiquement. C'est pourquoi, leur mise en contact avec la solution aqueuse d'acide alcanesulfonique à purifier doit être faite à une température n'excédant pas celle entraînant la dégradation de la résine. Cette dernière est en général inférieure à 120°C ; aussi le procédé selon l'invention est avantageusement réalisé en-dessous de 80°C et, de préférence, à la température ambiante.

On préfère utiliser des résines sous leur forme chlorure car, après la mise en contact avec l'acide alcanesulfonique à purifier et séparation par tout moyen connu approprié (en particulier par filtration, percolation, centrifugation,...), l'acide chlorhydrique formé par l'échange des anions sulfate et chlorure peut être facilement éliminé par strippage de la solution purifiée. Cette opération peut s'effectuer sous vide et à chaud (25 à 120°C, préférentiellement de 30 à 80°C), éventuellement avec addition de vapeur d'eau. Dans le cas de l'AMS, on peut ainsi obtenir un acide contenant moins de 100 ppm d'acide sulfurique et moins de 10 ppm d'acide chlorhydrique.

L'efficacité de la purification dépend évidemment du temps de mise en contact de la solution aqueuse d'acide alcanesulfonique avec la résine basique et de l'état de saturation de cette dernière. Rapporté au volume de liquide à traiter par rapport au volume de résine et par heure, le débit (bv) peut aller de 0,1 à 5 mais il est avantageux d'opérer à une valeur bv inférieure à 2,5 et, de préférence, au plus égale à 0,5.

Dans les exemples suivants qui illustrent l'invention sans la limiter, les pourcentages et ppm sont exprimés en poids. Le dispositif expérimental utilisé était constitué d'une pompe péristaltique alimentant, à partir d'un réservoir d'AMS à purifier, une colonne en verre contenant 20 ml de résine échangeuse d'anions et, à la sortie de cette colonne, d'un réceptacle pour l'AMS purifié.

### EXEMPLE 1

On a utilisé la résine DIAION® HPA25 sous forme de billes de 0,3-0,35 mm (taille effective) et de coefficient d'uniformité ≤1,5. Cette résine basique forte de porosité élevée à groupements triméthylammonium sous forme Cl^{⊖} présente les caractéristiques suivantes :
- squelette : copolymère styrène-divinylbenzène
- capacité totale d'échange : ≥ 0,6 eq/l
- rétention d'eau : 58-68 %
- température maximale d'utilisation: 100°C
- masse volumique : 630 g/l

La solution d'AMS traitée était un mélange AMS-Eau contenant 70 % d'AMS et environ 450 ppm d'acide sulfurique.

Les essais ont été réalisés à deux débits (bv) différents, à savoir 0,5 et 2,5 correspondant respectivement à 10 et 50 ml/h d'AMS à purifier.

Les résultats obtenus sont rassemblés dans le tableau suivant qui indique la concentration en acide sulfurique en fonction du volume (V en ml) d'AMS traité.

| **Vml** | **bv = 0,5** | **bv = 2,5** |
|---|---|---|
| | **[H**_{**2**}**SO**_{**4**}**] ppm** | **[H**_{**2**}**SO**_{**4**}**] ppm** |
| 10 | 20 | 19,5 |
| 20 | 22 | 29 |
| 30 | 25 | 51,2 |
| 40 | 35 | 86 |
| 50 | 71 | 131,7 |

### EXEMPLE 2

On a répété l'exemple 1 à un débit (bv) de 0,5 et en utilisant une solution d'AMS à 70 % contenant environ 1900 ppm d'acide sulfurique.

Le tableau suivant rassemble les résultats obtenus.

| | | | | | |
|---|---|---|---|---|---|
| V(ml) | 5 | 10 | 32 | 42 | 52 |
| [H₂SO₄] ppm | < 5 | 33 | 45 | 130 | 434 |

### EXEMPLE 3

On a répété l'exemple 1 à un débit (bv) de 2,5 et en remplaçant la résine DIAION® HPA25 par la résine AMBERLITE® IRA92. Cette résine faiblement basique à groupements dialkylamino présente les caractéristiques suivantes :
- squelette : polystyrène macroporeux
- capacité totale d'échange : ≥ 1,6 eq/l
- rétention d'eau : 40-50 %
- température maximale d'utilisation: 90°C
- masse volumique : 620-690 g/l
- aspect physique : billes de taille effective ≥ 450 µm et de coefficient d'uniformité ≤1,8

Les résultats sont rassemblés dans le tableau suivant

| | | | |
|---|---|---|---|
| V(ml) | 10 | 20 | 40 |
| [H₂SO₄]ppm | 18 | 65 | 151 |

## Revendications

1. Procédé de purification d'un acide alcanesulfonique pour en réduire la teneur en acide sulfurique et accessoirement celle d'anions autres que l'anion alcanesulfonate, **caractérisé en ce qu'**il comprend au moins une étape de mise en contact d'une solution aqueuse de l'acide alcanesulfonique à purifier avec une résine basique échangeuse d'anions.

2. Procédé selon la revendication 1 dans lequel la teneur en acide alcanesulfonique de la solution aqueuse à purifier est comprise entre 60 et 80 % et, de préférence 70 %.

3. Procédé selon la revendication 1 ou 2 dans lequel on utilise une résine à groupements fonctionnels ammonium quaternaire, de préférence sous la forme chlorure.

4. Procédé selon l'une des revendications 1 à 3 dans lequel la, mise en contact a lieu à une température n'excédant pas celle entraînant la dégradation de la résine, avantageusement à une température inférieure à 80°C et, de préférence, à la température ambiante.

5. Procédé selon l'une des revendications 1 à 4 dans lequel le débit horaire (bv) de liquide à traiter par volume de résine est compris entre 0,1 et 5, de préférence inférieur à 2,5 et avantageusement au plus égal à 0,5.

6. Procédé selon l'une des revendications 1 à 5 dans lequel, après la mise en contact, le produit résultant est soumis à une étape de strippage.

7. Procédé selon la revendication 6 dans lequel le strippage est réalisé sous vide et à une température allant de 25 à 120°C, préférentiellement de 30 à 80°C.

8. Procédé selon l'une des revendications 1 à 7 dans lequel l'acide alcanesulfonique à purifier contient de 1 à 12 atomes de carbone et est, de préférence, l'acide méthanesulfonique.

9. Procédé selon l'une des revendications 1 à 7 dans lequel on part d'une solution aqueuse à 70 % en poids d'acide méthanesulfonique.

## Patentansprüche

1. Verfahren zur Reinigung einer Alkansulfonsäure zur Verringerung deren Gehalts an Schwefelsäure und außerdem an anderen Anionen als das Alkalisulfonatanion, **dadurch gekennzeichnet, daß** das Verfahren mindestens einen Schritt umfaßt, bei dem eine wäßrige Lösung der zu reinigenden Alkansulfonsäure mit einem basischen Anionenaustauscherharz in Kontakt gebracht wird.

2. Verfahren nach Anspruch 1, wobei der Gehalt an Alkansulfonsäure der zu reinigenden wäßrigen Lösung zwischen 60 und 80 %, vorzugsweise 70 %, liegt.

3. Verfahren nach Anspruch 1 oder 2, wobei man ein mit quartären Ammoniumgruppen funktionalisiertes Harz, vorzugsweise in der Chloridform, verwendet.

4. Verfahren nach einem der Ansprüche 1 bis 3, wobei das Inkontaktbringen bei einer Temperatur, die nicht diejenige übersteigt, die zum Abbau des Harzes führt, vorzugsweise bei einer Temperatur kleiner als 80 °C und bevorzugt bei Umgebungstemperatur, durchgeführt wird.

5. Verfahren nach einem der Ansprüche 1 bis 4, wobei der stundenbezogene Durchsatz (Stundenleistung) (bv) der zu behandelnden Flüssigkeit pro Harzvolumen 0,1 bis 5, insbesondere weniger als 2,5 und vorzugsweise höchstens 0,5, beträgt.

6. Verfahren nach einem der Ansprüche 1 bis 5, wobei das resultierende Produkt nach dem Inkontaktbringen einem Abstreifschritt (Stripping) unterzogen wird.

7. Verfahren nach Anspruch 6, wobei das Abstreifen im Vakuum bei einer Temperatur von 25 bis 120 °C, vorzugsweise von 30 bis 80 °C, durchgeführt wird.

8. Verfahren nach einem der Ansprüche 1 bis 7, wobei die zu reinigende Alkansulfonsäure 1 bis 12 Kohlenstoffatome aufweist und vorzugsweise Methansulfonsäure ist.

9. Verfahren nach einem der Ansprüche 1 bis 7, wobei man von einer wäßrigen Lösung mit 70 Gew.-% Methansulfonsäure ausgeht.

## Claims

1. Process for the purification of an alkanesulphonic acid in order to reduce the content of sulphuric acid therein and, incidentally, that of anions other than the alkanesulphonate anion, **characterized in that** it comprises at least one stage in which an aqueous solution of the alkanesulphonic acid to be purified is brought into contact with a basic anion-exchange resin.

2. Process according to Claim 1, in which the content of alkanesulphonic acid in the aqueous solution to be purified is between 60 and 80% and preferably is 70%.

3. Process according to Claim 1 or 2, in which use is made of a resin comprising quaternary ammonium functional groups, preferably in the chloride form.

4. Process according to one of Claims 1 to 3, in which the contacting operation is carried out at a temperature not exceeding that resulting in decomposition of the resin, advantageously at a temperature of less than 80°C and preferably at room temperature.

5. Process according to one of Claims 1 to 4, in which the hourly throughput (bv) of liquid to be treated per volume of resin is between 0.1 and 5, preferably less than 2.5 and advantageously at most equal to 0.5.

6. Process according to one of Claims 1 to 5, in which, after the contacting operation, the resulting product is subjected to a stripping stage.

7. Process according to Claim 6, in which the stripping is carried out under vacuum and at a temperature ranging from 25 to 120°C, preferably from 30 to 80°C.

8. Process according to one of Claims 1 to 7, in which the alkanesulphonic acid to be purified comprises from 1 to 12 carbon atoms and is preferably methanesulphonic acid.

9. Process according to one of Claims 1 to 7, in which the starting material is an aqueous solution comprising 70% by weight of methanesulphonic acid.
